# EUROPEAN PATENT SPECIFICATION

(11) **EP 0 833 618 B1**
(45) Date of publication and mention of the grant of the patent: **10.04.2002**
(21) Application number: 96918678.2
(22) Date of filing: 05.06.1996
(51) Int. Cl.: A61K 9/20, A61K 9/48, A61K 9/50

(54) **SOLID PHARMACEUTICAL FORM FOR ORAL USE**
FESTE ORAL ANZUWENDENDE ARZENEIFORM
FORME PHARMACEUTIQUE SOLIDE A USAGE PAR VOIE ORALE

(30) Priority: 09.06.1995 IT MI951223
(43) Date of publication of application: 08.04.1998
(73) Proprietor: APR APPLIED PHARMA RESEARCH S.A., 6855 Stabio (CH)
(72) Inventor: CONTE, Ubaldo, I-27100 Pavia (IT); MAGGI, Lauretta, I-27100 Pavia (IT); REINER, Alberto, I-22100 Como (IT)
(74) Representative: Dragotti, Gianfranco
(86) International application number: EP9602495
(87) International publication number: WO9641617

(56) References cited:
- EP-A- 0 384 514
- WO-A-88/08299
- WO-A-93/05769
- WO-A-94/05277
- WO-A-94/06416
- WO-A-95/01781
- US-A- 5 338 550

## Description

The present invention relates to solid pharmaceutical forms which can be used principally orally and which are capable of releasing the active substance(s) contained therein in accordance with release kinetics which may advantageously be predetermined by means of suitable in vitro experiments.

More specifically, the invention relates to solid pharmaceutical forms, especially tablets, which are intended principally for the oral administration of active ingredients that are poorly soluble in water or in an acid medium and that are normally characterised by an erratic therapeutic effect because absorption is greatly limited by the solubility of the active substance.

The aim of the present invention is preferably to introduce into a carrier active ingredients, such as pain-killing, anti-inflammatory and anti-angina drugs, and, in general, other active ingredients which are required to exhibit not only an immediate therapeutic effect but also the maintenance of activity over a more prolonged period.

There is a great multiplicity of pharmaceutical forms permitting the administration of drugs which are to remedy acute clinical pictures, such as those marked by painful symptoms. A typical example is thus that of drugs having a pain-killing and/or anti-inflammatory activity, which will be the example principally referred to hereinafter, but it should be understood that this is in no way to be regarded as limiting.

One of the main requirements which must be met by a pharmaceutical form which contains an active ingredient having a pain-killing, anti-angina and analgesic activity in general is that it must bring about efficient levels of plasma rapidly and thus likewise the rapid onset of the desired therapeutic effect.

In most cases, oral administration involves a substantial delay in the onset of the therapeutic effect compared, for example, with parenteral administration, because the solid pharmaceutical form (capsule or tablet) has to undergo processes of disintegration and then dissolution of the active ingredient contained therein. In fact, only the dissolved or solubilised active substance may be absorbed because, by the absorption, levels of plasma result which, from a certain plasma concentration, can bring about the desired therapeutic effect.

Depending on the solubility of the active ingredient in water and/or in an acid medium, this series of processes may occur within very different times and in very different ways and thus the therapeutic efficacy often manifests itself after an excessively long period of time after the moment of administration: the entire series of processes in question generally indicated by the initials ADME (absorption, distribution, metabolism and elimination) and above all the time interval necessary for the occurrence of the first two (absorption and distribution) may impede or reduce drastically the therapeutic efficacy of a preparation intended to remedy acute painful symptoms.

As already mentioned, the onset of the therapeutic effect of an active substance administered orally is consequently greatly limited by the poor solubility of the drug in an aqueous medium or in gastric and intestinal fluids.

Many proposals and solutions have been advanced for overcoming this disadvantage, which is encountered, for example, with many analgesic and non-steroidal anti-inflammatory drugs (so-called NSAID), such as, for example, the preparation of cyclodextrin complexes or the preparation of active ingredients coated with or contained in suitable supports capable of improving the rate of dissolution and thus promoting absorption.

These complexes and derivatives, although they exhibit improved solubility and, at the same time, improved bioavailability characteristics, are nevertheless characterised by the fact that the formation of the complex generally involves the use of equimolar ratios between the active ingredient and the cyclodextrin or substantial amounts of excipients for promoting the solubilisation of the active ingredient.

These technological measures can bring about a substantial increase in the molecular weight and this weight increase can affect finished pharmaceutical forms which, with the same content of active ingredient, are difficult to administer and/or at any rate are not readily accepted by the patient.

In any case, the main demand made of a tablet and/or at any rate a solid pharmaceutical form for the oral administration of an analgesic drug is that its administration should bring about the rapid mitigation, if not the disappearance, of the painful symptom; in addition to that demand, another important requirement is that it should be possible to prolong the effect over time, thereby also permitting a substantial simplification of the daily dosage.

In the context of the solution to these problems, a large number of forms have been described and/or developed in the past, including:
- patent USA 2,951,792 (lipid matrix);
- British patent 2,230,185 (double-layered tablet);
- international patent application WO-A-88 08299;
- international patent application WO-A-95 01781 which describes a double-layered tablet of which the two layers contain different doses of the same active ingredient which is then released at quite different rates.
- international patent application WO-A-94 05277 describing a controlled release double-layered tablet which contains sodium naproxen in the immediate release layer and acid naproxen in the delayed release one, said acid naproxen layer comprising an immediate release granulate of acid naproxen and a delayed release granulate one compressed together, both layers being provided in a lipidic matrix.

It has now been found, and this forms the subject-matter of the present invention, that, by using a particular solid pharmaceutical form having two component fractions and by using novel measures in the formulation of an active ingredient which, as such, is poorly soluble in water, it is possible to obtain a solid pharmaceutical form which is characterised by:
- a rapid and complete disintegration of one of the two component fractions and by an equally rapid dissolution of the active ingredient contained in said fraction in the form of a derivative, for example a very soluble salt, which rapidly brings about high plasma levels and the consequent manifestation of the desired therapeutic effect;
- a slow and progressive disintegration or solubilisation of the second fraction of the pharmaceutical form and by a likewise slow and programmable process of dissolution of the active ingredient contained as such, that is to say, in non-derivative form, in the second fraction so as to bring about a prolonged and constant maintenance of efficient drug plasma levels and thus a prolonged therapeutic effect.

The novel pharmaceutical form is above all intended for the administration of an active ingredient that is to act immediately after administration and that is capable of maintaining the therapeutic efficacy for a prolonged period of time, ie. that is capable of bringing about immediately a high level in the blood and the consequent appearance of the therapeutic effect within a very limited time interval and of maintaining this efficient activity for a prolonged period of time.

The present invention is preferably used in the formulation of non-steroidal anti-inflammatory drugs and, more generally, for the administration of analgesic drugs exhibiting poor solubility.

By the use of established production techniques and novel formulation criteria as well as special excipients, the present invention also makes it possible to obtain solid pharmaceutical forms that can be used for oral administration and that are capable of releasing, completely and within a very short time interval, the active ingredient contained in soluble form therein, in order to obtain a high level of bioavailability, and of maintaining this therapeutic effect for a prolonged period because the poorly soluble active ingredient is released from the second fraction over a prolonged time interval.

The novel pharmaceutical form forming the subject-matter of the present invention is characterised in that, in the simplest case, it is formed by a system of two component fractions containing:
(a) in the first fraction, the active ingredient is contained in the form of a soluble derivative (salt, ester, amide or another pharmaceutically acceptable derivative) together with technological excipients and adjuvants, said adjuvants being selected from the group of the natural and/or synthetic and/or semisynthetic polymers belonging to the class of disintegrators and/or super-disintegrators, enabling the mass to be compressed and/or processed and ensuring an immediate release of the active ingredient (or active ingredients if more than one) contained in that fraction;
(b) in the second fraction, the same active ingredient in non-derivative form and characterised by a substantially lower solubility in water and/or aqueous liquids, together with technological excipients and adjuvants which, in this case too, enable the mass to be compressed but which are such that they modulate, in accordance with accurately programmable kinetics and rates, the release of the active substance(s) contained in that fraction.

The invention is therefore characterised in that the same pharmaceutical form contains a pair of active substances of which one component is in salt and/or derivative form or at any rate has a greater solubility, and one component is in non-salt form and is characterised by a limited solubility in water and/or aqueous liquids.

The present invention is also characterised in that, if the active substance has chiral centres in one or both of the fractions, the active ingredient may be contained in racemic form or in enantiomeric form, which is more active.

In the preparation of each of the two above-mentioned component fractions, in addition to the active ingredient and depending on its characteristics of solubility in water and/or aqueous liquids, polymeric substances capable of modulating, that is to say, accelerating or slowing down, the release of the active ingredient are also used.

The expression "solid pharmaceutical form having two component fractions" is to be understood as meaning both the double-layered tablets, in which each layer corresponds to one of the two component fractions, and also:
(i) hard gelatin capsules which contain (or into which are introduced) one or more tablets containing one or more active ingredients in salt (and thus soluble) form and one or more tablets containing that active ingredient in non-salt (and thus poorly soluble) form;
(ii) hard gelatin capsules containing one or more double-layered tablets prepared by the methods mentioned above;
(iii) hard gelatin capsules into which are introduced different granulates or pelletised systems containing one or more active ingredients, or alternatively the active ingredient in salt and thus soluble form and the same or another active ingredient in non-salt and thus poorly soluble form.

In the case of active ingredients that are poorly soluble in water, such as, for example, p-isobutylphenylpropionic acid (ibuprofen) which forms the subject of some of the examples which follow, the active ingredient contained in the first fraction (which disintegrates and dissolves rapidly) is in salt form, especially in the form of a lysine salt, while in the said second fraction (which disintegrates and dissolves slowly) the same active ingredient is used as such, that is to say, in non-salt form.

The described pharmaceutical form may contain active ingredients such as non-steroidal anti-inflammatory drugs (NSAlD) or steroidal anti-inflammatory drugs, sleep-inducing and tranquillising substances, substances having a preventive activity with respect to angina and hypertension attacks, or anti-histamine and anti-asthmatic drugs.

Bearing in mind the chemical characteristics of the active ingredient, it is possible to use salts (with acids, with organic or inorganic bases), esters, amides and in any case derivatives of which the solubility characteristics differ from those of the non-derivative active ingredient.

In the case of non-steroidal anti-inflammatory drugs, such as, for example, ibuprofen, naproxen, ketoprofen, indomethacin, acetylsalicylic acid, mefenamic acid, flufenamic acid, tiaprofenic acid, tolfenamic acid, diflunisal, sulindac, it is possible to use as soluble derivatives sodium and potassium salts of inorganic bases in general or of organic bases, or amino acids such as glycine, betaine, histidine, lysine, arginine, omithine, leucine, tyrosine, either in racemic or in optically active form.

Analogously, the active ingredients indicated above can also be used either in racemic form or in one of the more active diastereoisomeric forms.

In the preparation of the rapid-release first component fraction, it is possible to use customary pharmaceutical excipients, such as diluents, binders, disintegrators and all the other materials well known to the person skilled in the art.

As polymeric substances suitable for promoting an immediate disintegration of the rapid-release fraction of the active ingredient, it is possible to use so-called super-disintegrating excipients, such as, for example, crosslinked polyvinylpyrrolidone, crosslinked sodium carboxymethylcellulose, carboxymethyl starch, a copolymer of potassium methacrylate and divinylbenzene, polyvinyl alcohols, starches and their derivatives, beta-cyclodextrin and derivatives of dextrins in general. The polymeric substances constitute from 1.0 to 90% by weight of the fraction.

It is also possible to use in the first fraction other adjuvant substances formed by so-called effervescent mixtures, that is to say, substances capable of producing the rapid disintegration of the tablet or, in this specific case, the first layer, when the pharmaceutical form comes into contact with aqueous liquids and, preferably, with gastric juice.

These substances may include the carbonates and bicarbonates of sodium and of other alkali metals or alkaline earth metals, sodium carbonate glycocoll and other pharmaceutically acceptable salts capable of producing effervescence in an acid medium.

The adjuvants that may be used in the first fraction include pharmaceutically acceptable organic or inorganic substances having acidic or basic characteristics, or buffer mixtures, which are used to maintain, during the dissolution stage, a micromedium having an optimum pH value for the solubilisation of the active substance. It is also possible to use for that purpose amino acids, such as glycine, betaine, histidine, lysine, arginine, ornithine, leucine, tyrosine, either in racemic form or in an optically active form.

In the preparation of the second fraction which is released slowly and/or which is at any rate characterised by an active ingredient release rate which differs from that of the first fraction, it is possible to use natural and/or synthetic or semi-synthetic biocompatible polymeric materials belonging to the class of the so-called gelling and/or erodible hydrophilic polymers capable of slowing down the release of the active ingredient from the second layer.

Especially suitable for the preparation of the slow-release layer are cellulose derivatives and, to be more precise, those selected from the class consisting of hydroxypropylmethylcellulose having a molecular weight of from 1000 to 4,000,000 Daltons, hydroxypropylcellulose having a molecular weight of from 1,000 to 2,000,000 Daltons, carboxyvinyl polymer, polyvinyl alcohols, glucans, xanthans, mannans, galactomannans, scleroglucans, alginates, pectins, amylose with different degrees of crosslinking, carboxymethylcellulose and its derivatives, methylcellulose, ethylcellulose and their salts and/or derivatives and, in general, biocompatible and, preferably, biodegradable cellulose derivatives that are acceptable from the pharmaceutical point of view.

Different types of all the classes of polymer mentioned above are on the market and are at any rate available and are characterised by physico-chemical properties that differ especially in respect of solubility and gelling and/or erosion properties.

In the case of hydroxypropylmethylcellulose, it is possible to use various types having a molecular weight within the above-mentioned range and having different degrees of substitution.

These classes of hydroxypropylmethylcellulose exhibit different characteristics which is why they can encounter erosion and/or gelling processes on contact with aqueous liquids, which properties are associated with the viscosity exhibited by the polymeric material.

Depending on the solubility characteristics of the active ingredient, it is possible to use mainly gelling or erodible polymer derivatives, also depending on the desired release rate required for the active ingredient contained in the slow- release layer.

The quality and quantity of polymeric material can be determined on the basis of the results of in vitro experiments carried out by means of suitable tests.

The polymeric substances may be present in a percentage varying from 5 to 90% relative to the total weight of the slow-release layer, but preferably in a percentage varying from 30 to 75%.

It is possible to use as substances for limiting and/or at any rate delaying the release of the active ingredient from the second fraction, in association with or instead of the hydrophilic polymeric substances, hydrophobic excipients or lipophilic substances, such as glyceryl monostearate, glyceryl palmitostearate, glyceryl behenate, hydrogenated castor oil, solid derivatives of other pharmaceutically acceptable vegetable and/or animal oils, waxes, mono-, di-and tri-substituted glycerides and at any rate derivatives and fatty substances capable of modulating the release of the active substance.

Finally, it is possible to use in both fractions, for example, of double-layered tablets, excipients customarily used in pharmaceutical technology, such as mannitol, lactose, magnesium stearate, talcum, colloidal silica and others, such as glyceryl monostearate, stearic acid, hydrogenated castor oil, waxes, mono-, di- and tri-substituted glycerides and polyoxyethylene glycols having a molecular weight of from 400 to 50,000.

A film of entero-soluble polymeric material resistant to gastric juices may subsequently be applied to the finished tablets in order to permit the activation of the system only after the tablet has reached the duodeno-intestinal tract. With this method it is also possible to produce pharmaceutical forms intended for release in the intestine and/or colon.

It is possible to use as polymeric materials for obtaining enteric-coated systems: cellulose acetophthalate, cellulose acetopropionate, cellulose trimellitate, acrylic and methacrylic polymers and copolymers, having different molecular weights and different pH-dependent solubilities. The materials can be applied to the finished pharmaceutical form by means of the conventional film-coating method using solutions in organic solvents or aqueous dispersions and operating in a fluidised bed or vessel in accordance with known techniques.

The entero-soluble materials resistant to gastric juices can also be used in association with retarding polymers.

The Examples and the results obtained in the experimental forms described clarify the features and functionality of the novel form according to the invention.

In each case, the innovative nature of the embodiment is characterised by the fact that it is possible to obtain the claimed therapeutic system using established methods of production currently in use, that is to say, the system can be converted directly to an industrial scale.

The following Examples illustrate in non-limiting manner some solid pharmaceutical forms produced in accordance with the invention.

Example 1: Double-layered tablet containing 200 mg of ibuprofen of which a quantity corresponding to 100 mg (in the form of lysinised ibuprofen) is contained in the rapid-release first layer and 100 mg in the form of ibuprofen are contained in the second layer that is to release the active ingredient slowly.

### 1 - a - Preparation of the granulate which constitutes the rapid-release first layer containing ibuprofen in lysinised form as active ingredient. (Granulate A)

| | |
|---|---|
| lysinised ibuprofen (equivalent to 100.0 mg of ibuprofen) | 175.0 mg |
| corn starch (intragranular) | 40.0 mg |
| methylcellulose | 1.0 mg |
| corn starch (extragranular) | 20.0 mg |
| carboxymethyl starch | 16.0 mg |
| crosslinked polyvinylpyrrolidone | 6.0 mg |
| magnesium stearate | 3.2 mg |
| colloidal silica | 1.3 mg |
| total | 262.5 mg |

The lysinised ibuprofen and the first amount of corn starch (intragranular) are mixed and the mixture is wetted with a 1.3% aqueous methylcellulose solution until a homogeneously wetted mass is obtained. The moist mass is forced through a 25 mesh sieve and the granulate obtained is dried to constant weight in an oven and passed through the same sieve again. The second amount of corn starch (extragranular), the carboxymethyl starch, the crosslinked polyvinylpyrrolidone, the magnesium stearate and the colloidal silica are added and mixed in for 15 minutes in a Turbula.

A granulate is thus obtained which has good flow and compacting properties. The granulate is then subjected to the compression stage.

### 1 - b - Preparation of the granulate which constitutes the slow-release second layer containing ibuprofen in acid form as the active ingredient. (Granulate B)

| | |
|---|---|
| ibuprofen | 100.0 mg |
| mannitol hydroxypropylmethylcellulose | 30.0 mg |
| (Methocel K 15M) | 50.0 mg |
| polyvinylpyrrolidone | 15.0 mg |
| magnesium stearate | 2.0 mg |
| talcum | 3.0 mg |
| colloidal silica | 1.0 mg |
| total | 201.0 mg |

The ibuprofen, the mannitol and the hydroxypropylmethylcellulose are mixed and the mixture is wetted with a 10% polyvinylpyrrolidone solution until a homogeneously wetted mass is obtained. The moist mass is forced through a 25 mesh sieve and the granulate obtained is dried to constant weight in an oven and passed through the same sieve again. The magnesium stearate, the talcum and the colloidal silica are added and mixed in for 15 minutes in a Turbula.

A granulate is thus obtained having good flow and compacting properties. The granulate is then subjected to the compression stage.

### 1 - c - Preparation of the finished tablets

A Layer-press rotary tabletting machine (Manesty- Liverpool UK) or an equivalent machine capable of producing multi-layered tablets is used for the preparation of the tablets. As is known to the person skilled in the art, this machine is a rotary tabletting machine which has two or three charging stations and which is thus capable of producing two-layered or three-layered tablets; in this particular case, the machine is adjusted to produce two-layered tablets.

The machine used is equipped with oblong stamps (capsule-type of 16 x 6 mm).

The first charging hopper is filled with the granulate described at point 1-a (granulate A) while the second hopper is filled with the granulate described at point 1-b (granulate B).

The first charging station is adjusted to produce layers of 262.5 mg of granulate A (equivalent to 175 mg of lysinised ibuprofen, that is to say, equivalent to 100 mg of ibuprofen), while the second charging station is adjusted to provide a quantity of granulate B (with slow release of the active ingredient) of 201 mg (equivalent to 100 mg of ibuprofen).

By proceeding as described above, tablets having an average weight of 463.5 mg are obtained which contain a total of 200 mg of ibuprofen.

These finished systems are subjected to the dissolution test described below.

### 1 - d - Dissolution test (in vitro test) In order to evaluate the release characteristics of the finished systems, the apparatus 1, basket (described in USP XXII) is used, operating at 100 rpm and using as the dissolving fluid 1000 mL of simulated intestinal fluid (without enzymes) according to U.S.P. having a pH of 7.5 at 37oC.

The release of the active ingredient is monitored by U.V. spectrophotometric determination at 223 nm, using an automatic system of sampling and quantitative determination and an automatic programme for processing the data (Spectracomp 602 manufactured by Advanced Products-Milan).

The results of the experiments carried out are indicated in Table 1.

**TABLE 1**

| Time (min.) | mg of ibuprofen released |
|---|---|
| 5 | 101.2 |
| 15 | 108.9 |
| 30 | 117.2 |
| 60 | 132.1 |
| 120 | 151.2 |
| 240 | 178.5 |
| 360 | 202.8 |
| 420 | 203.4 |

It is clear that, of the 200 mg of active ingredient contained in the carrier, approximately 100 mg are released rapidly, within 15 minutes, while the second portion of 100 mg is released within approximately 6 hours.

### 1 - e - Bioavailability test (in vivo test on a healthy volunteer)

In order to evaluate the bioavailability characteristics of the active ingredient of the novel pharmaceutical form, the tablets described at point 1-c are administered to two healthy volunteers under rigorously controlled conditions.

The bioavailability of the active ingredient is followed by determining the plasma levels obtained, the values of which are indicated in Table 2 (mean values from the two volunteers).

**TABLE 2**

| Time | mcg/ml |
|---|---|
| 15 | 5.2 |
| 30 | 13.0 |
| 60 | 17.8 |
| 120 | 16.0 |
| 180 | 10.7 |
| 240 | 7.9 |
| 360 | 4.2 |
| 480 | 2.1 |
| 720 | 0.7 |

### Example 2: Comparative example

In order to evaluate the effective therapeutic efficacy of the invention a system analogous to that described in Example 1 was prepared for comparative purposes, but the layer described at point 1-a was replaced by a granulate containing the active ingredient ibuprofen in non-salt form and an identical formulation was used as regards technological excipients and adjuvants. Therefore the tablet involved is, especially, a double-layered tablet containing 200 mg of ibuprofen of which an amount corresponding to 100 mg of ibuprofen is contained in the rapid-release first layer and 100 mg of ibuprofen are contained in the second layer that is to release the active ingredient slowly (the second layer thus having the composition described at point 1-b).

### 2 - a - Preparation of the granulate which constitutes the rapid-release first layer containing 100 mg of ibuprofen as active ingredient. (Granulate C)

The composition of this granulate and the method of preparation are the same as those already indicated in Example 1, except that, instead of 175.0 mg of lysinised ibuprofen, 100.0 mg of ibuprofen are used.

### 2 - b - Preparation of the granulate which constitutes the slow-release second layer containing ibuprofen in acid form as the active ingredient.

Starting from the same composition, the same procedure as indicated at point 1-b of Example 1 is used.

### 2 - c - Preparation of the finished systems (by compression)

The procedure of Example 1-c is repeated.

By operating as described above, tablets having an average weight of 388.5 mg and containing a total of 200 mg of ibuprofen are obtained.

The finished tablets are subjected to the dissolution test.

### 2 - d - Dissolution test (in vitro test)

The experiment is carried out using the same apparatus and the same methods as those described above (Example 1-d).

The results of the experiments carried out are indicated in Table 3.

**TABLE 3**

| Time (min.) | mg of ibuprofen released |
|---|---|
| 5 | 8.7 |
| 15 | 24.5 |
| 30 | 52.6 |
| 60 | 84.2 |
| 120 | 105.8 |
| 180 | 124.6 |
| 240 | 142.1 |
| 360 | 172.4 |
| 420 | 183.1 |
| 480 | 196.7 |
| 600 | 203.5 |

It is clear that, of the 200 mg of active ingredient contained in the carrier, 100 mg are released within 90 to 120 minutes while the second portion of 100 mg is released within approximately 6 hours.

### 2 - e - Bioavailability test (in vivo test on a healthy volunteer)

The bioavailability test, followed by determining the resulting plasma levels, of the active ingredient of the tablets of Example 2-c was carried out by comparison by administering to the same subjects (after the necessary wash-out period) either the formulation described at point 1-c (Example 1) or that of Example 2-c, thus having an identical dosage of the active ingredient.

The results obtained are indicated in Table 4

**TABLE 4**

| Time | mcg/ml (Example 1) | mcg/ml (Example 2) |
|---|---|---|
| 15 | 5.2 | 0 |
| 30 | 13.0 | 7.8 |
| 60 | 17.8 | 13.1 |
| 120 | 16.0 | 10.1 |
| 180 | 10.7 | 8.0 |
| 240 | 7.9 | 6.5 |
| 360 | 4.2 | 4.2 |
| 480 | 2.1 | 1.9 |
| 720 | 0.7 | 0.9 |

The results indicated in the Table clearly demonstrate the more rapid generation of high plasma levels as a result of the administration of the pharmaceutical form containing the lysine salt of ibuprofen (Example 1) in the first layer compared with the comparative pharmaceutical form containing an identical amount of non-salt form ibuprofen (Example 2) in the first layer. In addition, the plasma concentrations obtained with the formulation of Example 1 remain high for a longer interval of time than do those obtained with the formulation of Example 2.

This result is entirely in line with the aims of the invention.

EXAMPLE 3: Double-layered tablet containing 500 mg of naproxen of which a quantity corresponding to 200 mg (in the form of betainated naproxen) is contained in the rapid-release first layer and 300 mg in the form of naproxen are contained in the second layer which is to release the active ingredient slowly.

### 3 - a - Preparation of the granulate which constitutes the rapid-release first layer containing naproxen in betainated form as active ingredient. (Granulate D)

| | |
|---|---|
| betainated naproxen (equivalent to 200.0 mg of naproxen) | 301.7 mg |
| corn starch (intragranular) | 40.0 mg |
| methylcellulose | 1.0 mg |
| corn starch (extragranular) | 20.0 mg |
| carboxymethyl starch | 16.0 mg |
| polyvinylpyrrolidone | 6.0 mg |
| magnesium stearate | 3.2 mg |
| colloidal silica | 1.3 mg |
| total | 389.2 mg |

The betainated naproxen and the first amount of corn starch (intragranular) are mixed and the mixture is wetted with a 1.3% aqueous methylcellulose solution until a homogeneously wetted mass is obtained. The moist mass is forced through a 25 mesh sieve and the granulate obtained is dried to constant weight in an oven and is passed through the same sieve again. The second amount of corn starch (extragranular), the carboxymethyl starch, the crosslinked polyvinylpyrrolidone, the magnesium stearate and the colloidal silica are added and mixed in for 15 minutes in a Turbula.

A granulate is thus obtained which exhibits good flow and compacting properties. The granulate is then subjected to the compression phase.

### 3 - b - Preparation of the granulate which constitutes the slow-release second layer containing naproxen in acid form (300 mg) as the active ingredient. (Granulate E)

| | |
|---|---|
| naproxen | 300.0 mg |
| mannitol hydroxypropylmethylcellulose | 30.0 mg |
| (Methocel K4M) | 50.0 mg |
| polyvinylpyrrolidone | 15.0 mg |
| magnesium stearate | 2.0 mg |
| talcum | 3.0 mg |
| colloidal silica | 1.0 mg |
| total | 401.0 mg |

The naproxen, the mannitol and the hydroxypropylmethylcellulose are mixed and wetted with a 10% polyvinylpyrrolidone solution until a homogeneously wetted mass is obtained. The moist mass is forced through a 25 mesh sieve and the granulate obtained is dried to constant weight in an oven and passed through the same sieve again.

The magnesium stearate, the talcum and the colloidal silica are added and mixed in for 15 minutes in a Turbula.

A granulate is thus obtained which exhibits good flow and compacting properties. The granulate is subjected to the compression stage as described above.

### 3 - c - Preparation of the finished systems (by compression)

The same procedure as that used in Example 1-c is used for the preparation of the tablets.

By operating as described above, tablets are obtained which have an average weight of 790.2 mg (389.2 mg constituting the first layer equivalent to 200 mg of naproxen and 401.0 mg constituting the second layer equivalent to 300 mg of naproxen) and which contain a total of 500 mg of naproxen and which are subjected to the dissolution test

### 3 - d - Dissolution test (in vitro test)

By proceeding as described in Example 1-d (using 1000 ml of distilled water as the dissolving fluid and operating at 265 nm in order to determine the active ingredient) the results indicated in Table 6 are obtained.

**TABLE 6**

| Time (min.) | mg of naproxen released |
|---|---|
| 5 | 82.5 |
| 15 | 202.3 |
| 30 | 253.6 |
| 60 | 337.8 |
| 120 | 380.4 |
| 240 | 445.1 |
| 360 | 485.0 |
| 420 | 507.2 |

It is clear that, of the 500 mg of active ingredient contained in the carrier, 200 mg are released rapidly, within 15 minutes, while the second portion of 300 mg is released within approximately 6-7 hours.

Example 4: Double-layered tablet containing 200 mg of R(+) ibuprofen of which an amount corresponding to 100 mg, in the form of R(+) sodium ibuprofen, is contained in the rapid-release first layer and 100 mg in the form of R(+) ibuprofen acid are contained in the second layer which is to release the active ingredient slowly.

### 4 - a - Preparation of the granulate which constitutes the rapid-release first layer (effervescent) containing R(+) sodium ibuprofen as the active ingredient. (Granulate F)

| | |
|---|---|
| R(+) ibuprofen sodium dihydrate (equivalent to 100.0 mg of ibuprofen) | 128.2 mg |
| corn starch | 40.0 mg |
| polyvinylpyrrolidone | 8.0 mg |
| citric acid | 20.0 mg |
| modified sodium bicarbonate | 10.0 mg |
| stearic acid | 3.0 mg |
| colloidal silica | 1.5 mg |
| total | 210.7 mg |

The R(+) sodium ibuprofen is mixed with the amount of corn starch and citric acid provided for and the whole is wetted with a 10% w/v polyvinyl-pyrrolidone solution in ethanol until a homogeneously wetted mass is obtained. The moist mass is forced through a 25 mesh sieve and the granulate obtained is dried to constant weight in an oven and is passed through the same sieve again. The modified sodium bicarbonate, the stearic acid and the colloidal silica are added and mixed in for 15 minutes in a Turbula.

A granulate is thus obtained which exhibits good flow and compacting properties. The granulate (granulate F) is then subjected to the compression stage.

### 4 - b - Preparation of the granulate constituting the second layer which has a release function and contains R(+) ibuprofen acid as the active ingredient. (Granulate G)

| | |
|---|---|
| R(+) ibuprofen acid | 100.0 mg |
| mannitol hydroxypropylmethylcellulose | 30.0 mg |
| (Methocel K15M) | 50.0 mg |
| polyvinylpyrrolidone | 15.0 mg |
| magnesium stearate | 2.0 mg |
| talcum | 3.0 mg |
| colloidal silica | 1.0 mg |
| total | 201.0 mg |

The R(+) ibuprofen acid, the mannitol and the hydroxypropylmethylcellulose are mixed and the whole is wetted with a 10% polyvinylpyrrolidone solution until a homogeneously wetted mass is obtained. The moist mass is forced through a 25 mesh sieve and the granulate obtained is dried to constant weight in an oven and passed through the same sieve again. The magnesium stearate, the talcum and the colloidal silica are added and mixed in for 15 minutes in a Turbula.

A granulate (granulate G) is thus obtained which exhibits good flow and compacting properties. The granulate is then subjected to the compression stage.

### 4 - c - Preparation of the finished systems (by compression)

By operating as described above (Example 1-c), tablets having an average weight of 411.7 mg and containing a total of 200 mg of R(+) ibuprofen are obtained and are subjected to the dissolution test.

### 4 - d - Dissolution test (in vitro test)

By operating as described in Example 1-d, the results indicated in Table 7 are obtained.

**TABLE 7**

| Time (min.) | mg of ibuprofen released |
|---|---|
| 5 | 68.9 |
| 15 | 97.8 |
| 30 | 116.5 |
| 60 | 124.7 |
| 120 | 147.0 |
| 240 | 169.4 |
| 360 | 190.6 |
| 420 | 204.6 |

It is clear that, of the 200 mg of active ingredient contained in the carrier, 100 mg are released rapidly, within 15 minutes, while the second portion of 100 mg is released within approximately 6 hours.

### Example 5 - Comparative test

In order to demonstrate the improvement and the greater effectiveness of the present invention over the compositions described in the already cited PCT application n. WO-A-94 05277, an in vivo comparative pharmacokinetic test has been carried out. For this purpose, a formulation was prepared according to the before described examples, consisting of a first immediate release layer containing sodium naproxen and a second delayed release layer containing naproxen. The formulation had the here-below reported composition:

| First (sodium naproxen) layer | |
|---|---|
| sodium naproxen | 235.50 mg (equivalent to 215 mg of naproxen) |
| corn starch (intragranular) | 43.02 mg |
| alluminum lake yellow | 0.02 mg |
| methylcellulose | 1.08 mg |
| sodium starch glycolate | 17.20 mg |
| crosslinked polyvinylpyrrolidone | 6.44 mg |
| corn starch (extragranular) | 21.50 mg |
| magnesium stearate | 3.44 mg |
| silicon dioxide | 1.40 mg |

| Second (naproxen) layer | |
|---|---|
| acid naproxen | 500.00 mg |
| hydroxypropylmethylcellulose | 50.00 mg |
| mannitol | 100.00 mg |
| polyvinylpyrrolidone | 16.70 mg |
| talc | 30.00 mg |
| magnesium stearate | 4.00 mg |
| silicon dioxide | 1.70 mg |

This pharmacokinetic test has been performed on three healty volunteers, the naproxen hematic levels being monitored over a period of 72 hours; the results are reported in table 8.

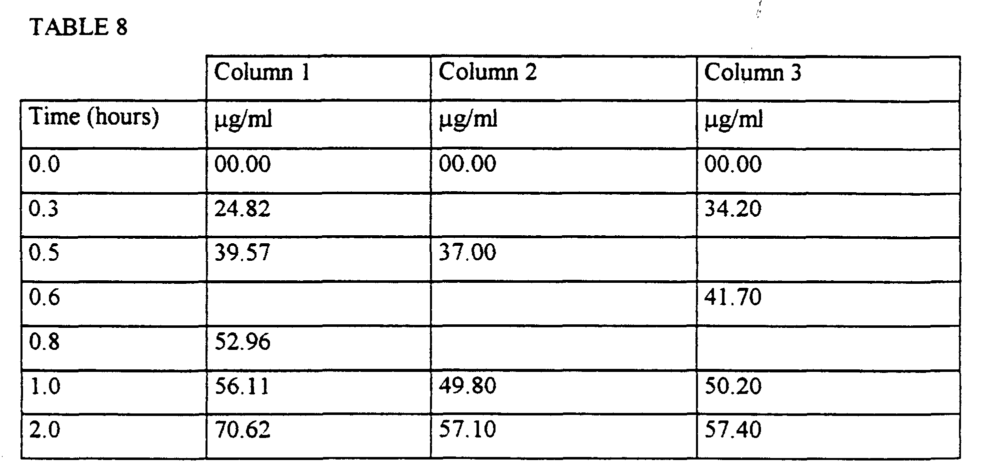

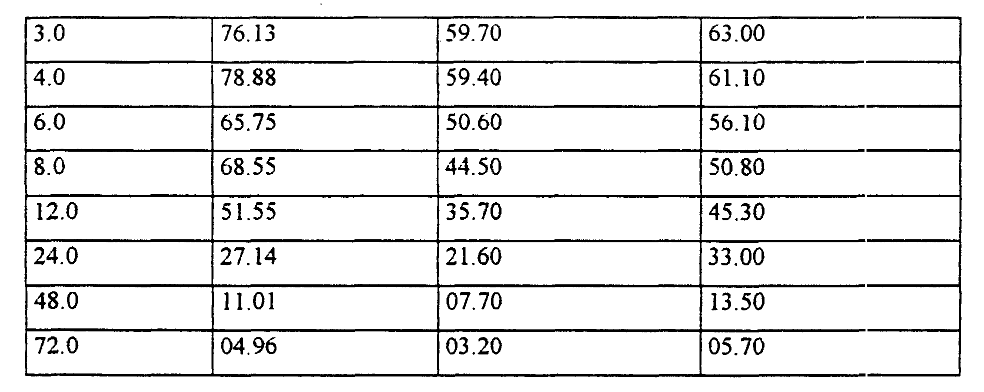

In column 1 it is reported the average of the values of the hematic levels of the three single volunteers; in column 2 and 3 the hematic levels of naproxen corresponding to the formulations respectively of examples 2 and 3 of the above cited PCT application are reported.

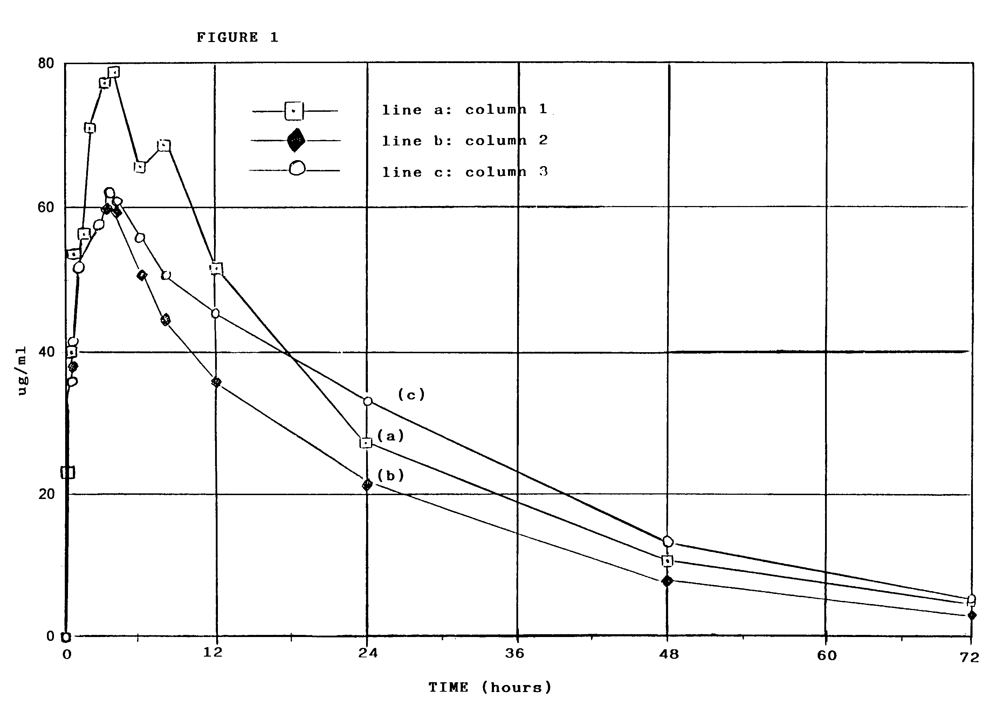

To better appreciate the differences between the formulation according to the present invention and those of the prior art, the above data have been transferred into fig. 1, from which it is clear that by using the formulation of the present invention higher immediate hematic naproxen levels can be obtained.

Moreover, it must be pointed out that the tablets according to the present invention, besides being more effective than those of the prior art, arc also manufactured by means of easier and more consolidated production technologies, with the consequent advantages from the economical point of view.

### Example 6

Comparative tests have also been carried out in order to show the greater effectivenes of the formulations of the present invention if compared to those commercially available. For this purpose a tablet, Ibuprofen based has been prepared, having the here-below indicated composition.

| Inbuprofen sodium layer | |
|---|---|
| sodium ibuprofenen | 336.00 mg (equivalent to 300 mg of ibuprofen) |
| methylcellulose | 1.05 mg |
| starch | 150.00 mg |
| sodium lauryl sulphate | 0.75 mg |
| crosslinked polyvinylpyrrolidone | 9.00 mg |
| dye (E 124) | 0.15 mg |
| sodium carboxymethylstarch | 24.00 mg |
| magnesium stearate | 4.05 mg |
| colloidal silica | 2.00 mg |
| cellulose (Avicel PH 101) | 62.00 mg |
| calcium carbonate | 30.00 mg |
| talc | 20.00 mg |

| Ibuprofen layer | |
|---|---|
| acid ibuprofen (micronized) | 500.00 mg |
| methylcellulose | 50.00 mg |
| mannitol | 100.00 mg |
| polyvinylpyrrolidone | 16.70 mg |
| talc | 35.00 mg |
| magnesium stearate | 8.04 mg |
| colloidal silica | 1.07 mg |
| polyethylenglycol | 5.00 mg |

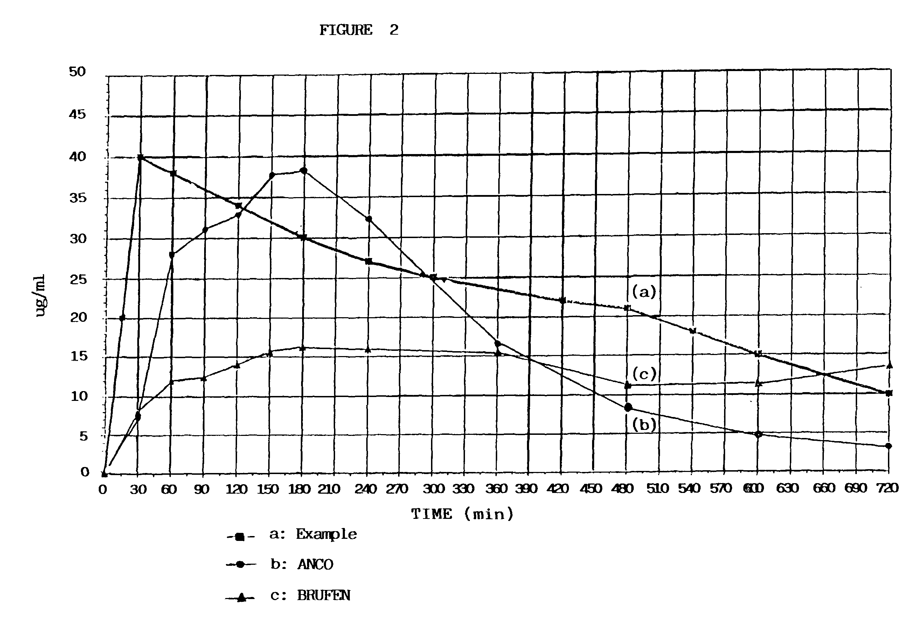

A pharmacokinetic test has then been carried out comparing the hematic levels of Ibuprofen obtained with the above-described tablets with those of two Ibuprofen containing tablets normally available in Switzerland (Brufen ®) and in germany (Anco ®) and having the same Ibuprofen content (800 mg). The results are reported in figure 2 as the mean values of the hematic levels of three single volunteers. From figure 2 it is possible to see how higher is the immediate bioavailability of Ibuprofen which can be obtained with the tablets of the present invention.

### Example 7

Another comparative test, as described in the previous example, has also been conducted with tablets having the here-below reported composition.

| Ibuprofen sodium layer | |
|---|---|
| sodium ibuprofen | 504.00 mg (equivalent to 450 mg of ibuprofen) |
| methylcellulose | 1.60 mg |
| starch | 220.00 mg |
| sodium lauryl sulphate | 1.12 mg |
| polyvinylpyrrolidone XL | 13.50 mg |
| sodium carboxymethylstarch | 36.00 mg |
| magnesium stearate | 6.00 mg |
| cellulose (Avicel PH 101) | 93.00 mg |
| talc | 10.00 mg |

| Ibuprofen layer | |
|---|---|
| acid ibuprofen | 50.00 mg |
| methylcellulose | 5.00 mg |
| lactose | 10.00 mg |
| polyvinylpyrrolidone | 1.60 mg |
| talc | 3.50 mg |
| magnesium stearate | 0.80 mg |
| polyethylenglycol | 0.50 mg |

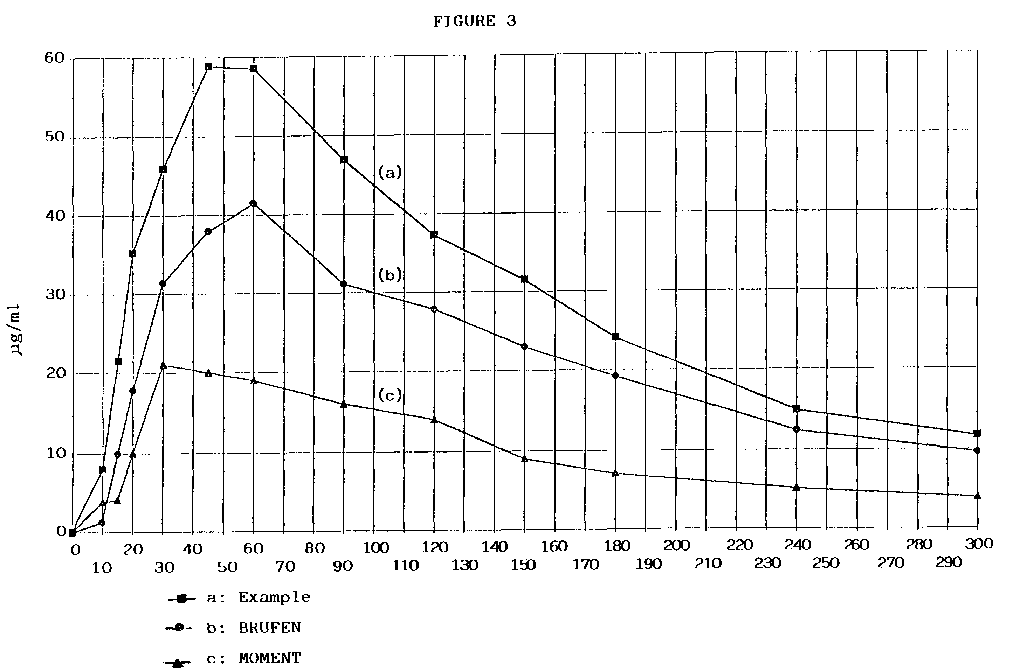

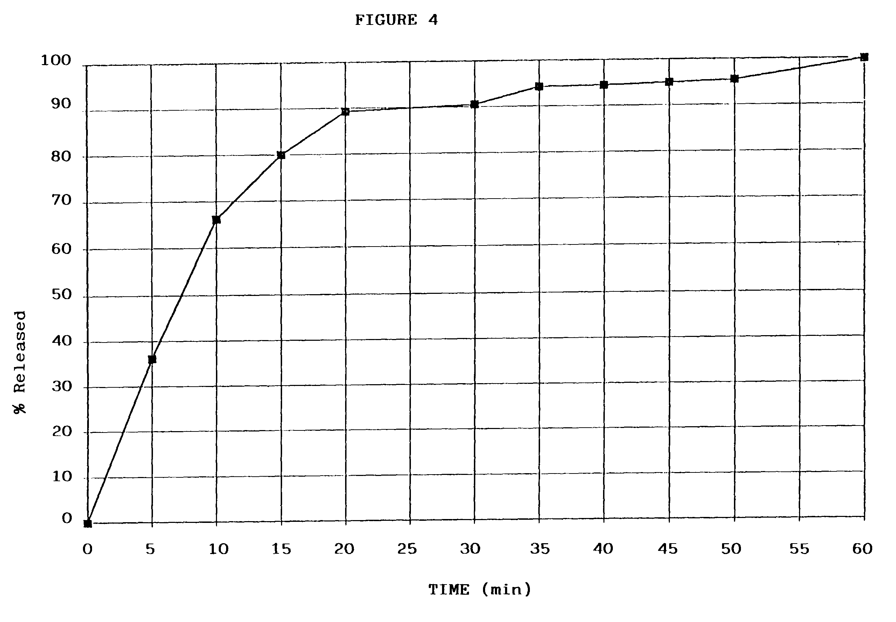

The hematic levels have been compared to those obtained using tablets normally available on the italian market (Brufen ® and Moment ®); from the result reported in figure 3 the same conclusions can be made. In figure 4 it is also reported the result of a dissolution test

It is clear that, if an active ingredient is soluble in the form in which it is normally available or obtained, it will be used as such in the first portion, that is to say, that intended for immediate release, while in the programmed slow-release second fraction it will be used in the form of a less soluble derivative or with one of the formulations known for prolonged release.

Finally, it should be noted that the above examples refer to double-layered tablets which constitute the preferred embodiment of the invention, it being understood that other embodiments are provided for, such as those mentioned above and especially capsules containing both the above-mentioned fractions.

## Claims

1. Solid pharmaceutical form for the oral administration of at least one active ingredient, with release of the latter at successive times and at different rates, **characterised in that** for each active ingredient it consists of two fractions only, wherein:
(a) in the first fraction, the active ingredient is contained in the form of a soluble derivative together with technological excipients and adjuvants, said adjuvants being selected from the group of the natural and/or synthetic and/or semi-synthetic polymers belonging to the class of disintegrators and/or super-disintegrators, enabling the mass to be compressed and/or processed and ensuring an immediate release of the active ingredient (or active ingredients if more than one) contained **in that** fraction;
(b) in the second fraction, the same active ingredient is contained in non-derivative form, **characterised by** a substantially lower solubility in water and/or aqueous liquids, together with technological excipients and adjuvants which, in this case too, enable the mass to be compressed but which are such that they modulate, in accordance with accurately programmable kinetics and rates, the release of the active ingredient(s) contained in that fraction.

2. Solid pharmaceutical form according to Claim 1, **characterised in that** the said at least one active ingredient is selected from:
(i) steroidal and non-steroidal anti-inflammatory drugs;
(ii) sleep-inducing and tranquillising substances;
(iii) substances active in the prevention of angina attacks and hypertension attacks;
(iv) anti-histamine and anti-asthmatic substances.

3. Solid pharmaceutical form according to Claim 2, **characterised in that** the anti-inflammatory drug is selected from ibuprofen, ketoprofen, acetylsalicylic acid, mefenamic acid, flufenamic acid, tiprofenic acid, tolfenamic acid, diflunisal, piroxicam, naproxen, flurbiprofen, sodium diclofenac and indomethacin.

4. Solid pharmaceutical form according to Claim 2, **characterised in that** the salts of sodium, potassium or inorganic or organic bases of the anti-inflammatory compounds are used as the soluble derivatives of those compounds.

5. Solid pharmaceutical form according to Claim 4, **characterised in that** the derivatives are glycine, betaine, histidine, lysine, arginine, ornithine, leucine, tyrosine salts either in racemic form or in optically active form.

6. Solid pharmaceutical form according to Claim 2, **characterised in that** the sleep-inducing and tranquillising substance is selected from diazepam, nitrazepam, clonazepam and oxazepam.

7. Solid pharmaceutical form according to Claim 2, **characterised in that** the anti-angina and anti-hypertensive substance is selected from diltiazem, urapidil, trapidil benziodaron and dipiridamol.

8. Solid pharmaceutical form according to Claim 2, **characterised in that** the anti-histamine or anti-asthmatic substance is selected from terfenadin, chlorpheniramine, terbutaline and combinations thereof.

9. Solid pharmaceutical form according to Claim 1, **characterised in that** said polymers are selected from crosslinked polyvinylpyrrolidone, crosslinked sodium carboxymethylcellulose, carboxymethyl starch, a copolymer of potassium methacrylate and divinylbenzene, polyvinyl alcohols, starches and their derivatives, microcrystalline cellulose and derivatives of cellulose, beta-cyclodextrin and derivatives of dextrin in general.

10. Solid pharmaceutical form according to Claim 9, **characterised in that** the polymers are present in the first fraction in an amount of from 1.0 to 90% by weight based on the weight of the fraction.

11. Solid pharmaceutical form according to Claim 10, **characterised in that** the polymers are present in the first fraction in an amount of from 20 to 70% by weight based on the weight of the fraction.

12. Solid pharmaceutical form according to Claim 1, **characterised in that** the adjuvant substances acting as diluents are selected, depending on the hydrophilic characteristics of the active ingredient, from the group consisting of starch, pregelled starch, calcium phosphate, mannitol, lactose, saccharose, glucose, sorbitol and microcrystalline cellulose.

13. Solid pharmaceutical form according to Claim 1, **characterised in that** the adjuvant substances acting as agglutinants are selected from the group of polymers having a binding power.

14. Solid pharmaceutical form according to Claim 13, **characterised in that** the polymers are selected from gelatin, polyvinylpyrrolidone, methylcellulose, sizing starch, ethylcellulose, gum arabic and gum tragacanth.

15. Solid pharmaceutical form according to Claim 1, **characterised in that** the adjuvant substances having the function of promoting the wettability and the solubilisation of the active ingredient are selected from the group consisting of ionic and non-ionic surfactants, saponins and sterols.

16. Solid pharmaceutical form according to Claim 15, **characterised in that** the substance promoting the wettability and solubilisation of the active ingredient is contained in a percentage of from 0.01 to 25% by weight based on the active ingredient.

17. Solid pharmaceutical form according to Claim 1, **characterised in that** the adjuvant substances acting as lubricants and anti-adherents are selected from the group consisting of magnesium stearate, stearic acid, colloidal silica, glyceryl monostearate, polyoxyethylene glycols having a molecular weight of from 400 to 50000, hydrogenated castor oil, waxes and mono-, di- and tri-substituted glycerides.

18. Solid pharmaceutical form according to Claim 1, **characterised in that** the adjuvant substances have an effervescent effect and are selected from the group consisting of carbonate of sodium and/or other alkali metals and/or alkaline earth metals, sodium bicarbonate, sodium carbonate glycocoll and other pharmaceutically acceptable salts capable of producing effervescence in an acid medium, citric acid, tartaric acid and fumaric acid.

19. Solid pharmaceutical form according to Claim 1, **characterised in that** the adjuvant substances producing effervescence are selected from citric acid, tartaric acid and fumaric acid.

20. Solid pharmaceutical form according to Claims 18 and 19, **characterised in that** the adjuvant substances having an effervescent effect are present in an amount of from 3 to 40% by weight of the weight of the first fraction.

21. Solid pharmaceutical form according to Claim 1, **characterised in that** the second fraction contains natural and/or synthetic or semi-synthetic biocompatible polymeric materials belonging to the class of the so-called gelling and/or erodible hydrophilic polymers capable of slowing down the release of the active ingredient from the second fraction.

22. Pharmaceutical form according to Claim 21, **characterised in that** the hydrophilic polymers are selected from the class consisting of hydroxypropylmethylcellulose having a molecular weight of from 1000 to 2,000,000 Daltons, carboxyvinyl polymer, polyvinyl alcohols, glucans, xanthans, mannans, galactomannans, scleroglucans, alginates, pectin, amylose having different degrees of crosslinking, carboxymethylcellulose and its derivatives, methylcellulose, ethylcellulose and their salts and/or derivatives.

23. Pharmaceutical form according to Claims 21 and 22, **characterised in that** the polymeric substances may be present in a percentage varying from 5 to 90% relative to the total weight of the slow-release fraction.

24. Pharmaceutical form according to Claims 21 and 22, **characterised in that** the polymeric substances may be present in a percentage varying from 30 to 75% relative to the total weight of the slow-release fraction.

25. Solid pharmaceutical form according to Claim 1, **characterised in that** it is in the form of a double-layered tablet of which each layer constitutes one of the component fractions.

26. Solid pharmaceutical form according to Claim 25, **characterised in that** it contains from 1 to 90% by weight of active ingredient.

27. Solid pharmaceutical form according to Claim 25, **characterised in that** it contains ibuprofen as the active ingredient, with a dimensional distribution of less than 20 microns.

28. Solid pharmaceutical form according to Claim 25, **characterised in that** it contains sodium lauryl sulphate as the surfactant substance.

29. Solid pharmaceutical form according to Claim 1, **characterised in that** it is in the form of a hard gelatin capsule which contains one or more tablets containing at least one active ingredient in a form soluble in water and aqueous liquids and one or more tablets containing the same at least one active ingredient in delayed and programmed release form.

30. Solid pharmaceutical form according to Claim 1, **characterised in that** it is in the form of a hard gelatin capsule which contains one or more double-layered tablets according to Claim 26.

31. Solid pharmaceutical form according to Claim 1, **characterised in that** it is in the form of a hard gelatin capsule which contains at least two different granulates of which at least one contains at least one active ingredient in a form readily soluble in water or aqueous liquids and at least one contains the same at least one active ingredient in a delayed and programmed release form.

32. Solid pharmaceutical form according to Claim 31, **characterised in that** the granulates are in pelletised form.

## Patentansprüche

1. Feste pharmazeutische Form zur oralen Verabreichung mindestens eines aktiven Bestandteils, wobei letzterer zu bestimmten Zeiten und bei unterschiedlichen Raten freigegeben wird,
**dadurch gekennzeichnet,**
**dass** sie für jeden aktiven Bestandteil aus nur zwei Fraktionen besteht, wobei:
a) in der ersten Fraktion der aktive Bestandteil in Form eines löslichen Derivates zusammen mit Trägern und Hilfsstoffen enthalten ist, wobei die Hilfsstoffe ausgewählt sind aus der Gruppe natürlicher und/oder synthetischer und/oder semi-synthetischer Polymere, die zur Klasse der Desintegratoren und/oder Super-Desintegratoren gehören, die das Komprimieren und/oder Bearbeiten der Masse ermöglichen und eine sofortige Freigabe des in der Fraktion enthaltenden aktiven Bestandteils (oder der aktiven Bestandteile bei mehr als einem) sicherstellen,
(b) in der zweiten Fraktion der gleiche aktive Bestandteil nicht in Form eines Derivats, und durch eine wesentlich niedrigere Löslichkeit in Wasser und/oder wässrigen Flüssigkeiten gekennzeichnet ist, zusammen mit Trägern und Hilfsstoffen enthalten ist, die in diesem Fall auch das Komprimieren der Masse ermöglichen aber die derart sind, dass sie gemäß der genau programmierbaren Kinetik und Raten die Freigabe der aktiven Bestandteile, die in der Fraktion enthalten sind, einstellen.

2. Feste pharmazeutische Form nach Anspruch 1, **dadurch gekennzeichnet, dass** der mindestens eine aktive Bestandteil ausgewählt ist aus:
(i) steriodalen und nicht-steriodalen anti-entzündungshemmenden Medikamenten,
(ii) schlafauslösenden und beruhigenden Substanzen,
(iii)Substanzen, die bei der Vorbeugung von Aginaattacken und Bluthochdruckattacken aktiv sind,
(iv) Antihistamin- und Antiasthma-Substanzen.

3. Feste pharmazeutische Form nach Anspruch 2, **dadurch gekennzeichnet, dass** das anti-entzündungshemmende Medikament ausgewählt ist aus Ibuprofen, Ketoprofen, Acteylsalicylsäure, Mefenamsäure, Flufenamsäure, Tiprofensäure, Tolfenamsäure, Diflunisal, Piroxikam, Naproxen, Flurobiprofen, Natrium-Diclofenac und Indomethacin.

4. Feste pharmazeutische Form nach Anspruch 2, **dadurch gekennzeichnet, dass** Natrium-, Kaliumsalze, anorganische oder organische Basen der anti-entzündungshemmenden Verbindungen als lösliche Derivate dieser Verbindungen verwendet werden.

5. Feste pharmazeutische Form nach Anspruch 4, **dadurch gekennzeichnet, dass** die Derivate Glycin, Betain, Histidin, Lysin, Arginin, Ornithin, Leucin, Tyrosin Salze entweder in der Form des Racemates oder in optisch aktiver Form vorliegen.

6. Feste pharmazeutische Form nach Anspruch 2, **dadurch gekennzeichnet, dass** die schlafauslösende und beruhigende Substanz ausgewählt ist aus Diazepam, Nitrazepam, Clonazepam und Oxazepam.

7. Feste pharmazeutische Form nach Anspruch 2, **dadurch gekennzeichnet, dass** die Anti-Angina- und Anti-Bluthochdruck-Substanz ausgewählt ist aus Diltiazem, Urapidil, Trapidil, Benziodaron und Dipiradamol.

8. Feste pharmazeutische Form nach Anspruch 2, **dadurch gekennzeichnet, dass** die Anti-Histamin- oder Anti-Asthma-Substanz ausgewählt ist aus Terfenadin, Chlorpheniramin, Terbutalin und deren Kombinationen.

9. Feste pharmazeutische Form nach Anspruch 1, **dadurch gekennzeichnet, dass** die Polymere ausgewählt sind aus vernetztem Polyvinylpyrrolidon, vernetzter Natriumcarboxymethylcellulose, Carboxymethylstärke, einem Polymer aus Kaliummethacrylat und Divinylbenzol, Polyvinylalkoholen, Stärken und deren Derivaten, mikrokristalliner Zellulose und Derivate von Zellulose, Beta-Cyclodextrin und Derivate von Dextrinen im allgemeinen.

10. Feste pharmazeutische Form nach Anspruch 9, **dadurch gekennzeichnet, dass** die Polymere in der ersten Fraktion in einer Menge von 1,0 bis 90 Gew.% auf Basis der Fraktion vorhanden sind.

11. Feste pharmazeutische Form nach Anspruch 10, **dadurch gekennzeichnet, dass** die Polymere in der ersten Fraktion in einer Menge von 20 bis 70 Gew.% auf Basis der Fraktion vorhanden sind.

12. Feste pharmazeutische Form nach Anspruch 1, **dadurch gekennzeichnet, dass** die Hilfsstoffe, die als Verdünner wirken, abhängig von den hydrophilen Eigenschaften des aktiven Bestandteils ausgewählt werden aus der Gruppe bestehend aus Stärke, vorgelierter Stärke, Calciumphosphat, Mannitol, Lactose, Saccharose, Glucose, Sorbitol und mikrokristalliner Zellulose.

13. Feste pharmazeutische Form nach Anspruch 1, **dadurch gekennzeichnet, dass** die Hilfsstoffe, die als Bindemittel wirken, ausgewählt sind aus der Gruppe von Polymeren, die ein Bindungspulver aufweisen.

14. Feste pharmazeutische Form nach Anspruch 13, **dadurch gekennzeichnet, dass** die Polymere ausgewählt sind aus Gelatine, Polyvinylpyrrolidon, Methylcellulose, Schlichtestärke, Ethylcellulose, Gummi-Arabicum und Tragacanth-Gummi.

15. Feste pharmazeutische Form nach Anspruch 1, **dadurch gekennzeichnet, dass** die Hilfsstoffe, die die Benetzbarkeit und Solubilisierung des aktiven Bestandteils fördern, ausgewählt sind aus der Gruppe bestehend aus ionischen und nicht-ionischen oberflächenaktiven Mitteln, Saponinen und Sterolen.

16. Feste pharmazeutische Form nach Anspruch 15, **dadurch gekennzeichnet, dass** die Substanz, die die Benetzbarkeit und Solubilisierung des aktiven Bestandteils fördert, in einem Prozentgehalt von 0,01 bis 25 Gew.% auf Basis des aktiven Bestandteils enthalten ist.

17. Feste pharmazeutische Form nach Anspruch 1, **dadurch gekennzeichnet, dass** die Hilfsstoffe, die als Gleitsubstanzen und Antihaftmittel wirken, ausgewählt sind aus der Gruppe bestehend aus Magnesiumstearat, Stearinsäure, colloidalem Siliziumdioxyd, Glycerol Monostearat, Polyoxyethylen Glycolen mit einem Molekulargewicht von 400 bis 50000, hydriertem Rizinusöl, Wachsen und mono-, di- und tri-substituierten Glyceriden.

18. Feste pharmazeutische Form nach Anspruch 1, **dadurch gekennzeichnet, dass** die Hilfsstoffe, die eine schäumende Wirkung haben, ausgewählt sind aus der Gruppe bestehend aus Natriumcarbonat und/oder anderen Alkalimetallen und/oder alkalischen Erdmetallen, Natriumbicarbonat, Natriumcarbonat, Glycol und anderen pharmazeutisch annehmbaren Salzen, die die Schaumherstellung in einem sauren Medium, in Zitronensäure, Tartarsäure und Fumarinsäure ermöglichen.

19. Feste pharmazeutische Form nach Anspruch 1, **dadurch gekennzeichnet, dass** die Hilfsstoffe, die Schaum erzeugen, ausgewählt sind aus Zitronensäure, Tartarsäure und Fumarinsäure.

20. Feste pharmazeutische Form nach den Ansprüchen 18 und 19, **dadurch gekennzeichnet, dass** die Hilfsstoffe, die eine schäumende Wirkung haben, in einer Menge von 3 bis 40 Gew.% des Gewichts der ersten Fraktion vorhanden sind.

21. Feste pharmazeutische Form nach Anspruch 1, **dadurch gekennzeichnet, dass** die zweite Fraktion natürliche und/oder synthetische oder semi-synthetische biokompatible Polymermaterialien, die zur Klasse der sogenannten gelbildenden und/oder erosionsempfindlichen hydrophilen Polymeren gehört, die die Abgabe des aktiven Bestandteils aus der zweiten Fraktion verlangsamen, enthält.

22. Pharmazeutische Form nach Anspruch 21, **dadurch gekennzeichnet, dass** die hydrophilen Polymere ausgewählt sind aus der Klasse bestehend aus Hydroxypyrrolmethylcellulose mit einem Molekulargewicht von 1000 bis 2000000 Daltons, Carboxyvinylpolymer, Polyvinylalkohol, Glucanen, Xanthanen, Mannanen, Galactomannanen, Scleroglucanen, Alginaten, Pectin, Amylose mit unterschiedlichen Vernetzungsgraden, Carboxymethylcellulose und dessen Derivaten, Methylcellulose, Ethylcellulose und dessen Salzen und/oder Derivaten.

23. Pharmazeutische Form nach den Ansprüchen 21 und 22, **dadurch gekennzeichnet, dass** die Polymersubstanz in einem Prozentgehalt vorhanden ist, der zwischen 5 bis 90 % im Verhältnis zum Gesamtgewicht der langsam abgebenden Fraktion variiert.

24. Pharmazeutische Form nach den Ansprüchen 21 und 22, **dadurch gekennzeichnet, dass** die Polymersubstanzen in einem Prozentgehalt vorhanden sind, der zwischen 30 bis 45 % im Verhältnis zum Gesamtgewicht der langsam abgebenden Fraktion variiert.

25. Feste pharmazeutische Form nach Anspruch 1, **dadurch gekennzeichnet, dass** sie in der Form einer doppelt beschichteten Tablette vorliegt, wobei jede Schicht aus einer der Komponentenfraktionen besteht.

26. Feste pharmazeutische Form nach Anspruch 25, **dadurch gekennzeichnet, dass** sie 1 bis 90 Gew.% des aktiven Bestandteils enthält.

27. Feste pharmazeutische Form nach Anspruch 25, **dadurch gekennzeichnet, dass** sie Ibuprofen als aktiven Bestandteil mit einer dimensionalen Verteilung von weniger als 20 Mikron enthält.

28. Feste pharmazeutische Form nach Anspruch 25, **dadurch gekennzeichnet, dass** sie Natriumlaurylsulphat als oberflächenaktive Substanz enthält.

29. Feste pharmazeutische Form nach Anspruch 1, **dadurch gekennzeichnet, dass** sie in der Form einer harten Gelatinekapsel vorliegt, die eine oder mehrere Tabletten enthält, die mindestens einen aktiven Bestandteil in einer Form, die in Wasser und wässrigen Flüssigkeiten löslich ist, enthält und eine oder mehrere Tabletten enthält, die mindestens den gleichen aktiven Bestandteil in einer verzögerten und programmierten Freigabeform enthält.

30. Feste pharmazeutische Form nach Anspruch 1, **dadurch gekennzeichnet, dass** sie in Form einer harten Gelatinekapsel vorliegt, die ein oder mehrere doppelbeschichtete Tabletten gemäß Anspruch 26 enthält.

31. Feste pharmazeutische Form nach Anspruch 1, **dadurch gekennzeichnet, dass** sie in der Form einer harten Gelatinekapsel vorliegt, die mindestens zwei verschiedene Granulate enthält, von denen mindestens eines mindestens einen aktiven Bestandteil in einer Form enthält, die leicht in Wasser oder wässrigen Lösungsmitteln löslich ist, und mindestens eines den gleichen mindestens einen aktiven Bestandteil in einer verzögerten und programmierten Freigabeform enthält.

32. Feste pharmazeutische Form nach Anspruch 31, **dadurch gekennzeichnet, dass** die Granulate in pelletierter Form vorliegen.

## Revendications

1. Forme pharmaceutique solide pour administration orale d'au moins un ingrédient actif avec libération de ce dernier à des moments successifs et à des vitesses différentes, **caractérisée par le fait que** pour chaque ingrédient actif elle consiste en deux fractions uniquement, dans lesquelles :
a) dans la première fraction, l'ingrédient actif est contenu sous la forme d'un dérivé soluble associé à des excipients et à des adjuvants technologiques, lesdits adjuvants étant choisis parmi le groupe des polymères naturels et/ou synthétiques et/ou semi-synthétiques faisant partie de la classe des délitants ou des super-délitants, permettant la compression et/ou le traitement de la masse et assurant la libération immédiate de l'ingrédient actif (ou des ingrédients actifs s'il y en a plusieurs) contenu(s) dans cette fraction ;
b) dans la seconde fraction, le même ingrédient actif est contenu sous une forme non dérivée, **caractérisée par** une solubilité considérablement moindre dans l'eau et/ou les fluides aqueux, associée à des excipients et des adjuvants technologiques qui, dans ce cas également, permettent la compression de la masse mais qui sont tels qu'ils modulent, conformément à une cinétique et des vitesses exactement programmables, la libération du ou des ingrédient(s) actif(s) contenus dans cette fraction.

2. Forme pharmaceutique solide selon la revendication 1 **caractérisée par le fait que** le dit au moins un ingrédient actif est choisi parmi les suivants :
(i) anti-inflammatoires stéroïdiens et non-stéroïdiens ;
(ii) substances somnifères et tranquillisantes ;
(iii) substances actives dans la prévention des crises d'angor et des crises d'hypertension ;
(iv) substances antihistaminiques et antiasthmatiques.

3. Forme pharmaceutique solide selon la revendication 2 **caractérisée par le fait que** le médicament anti-inflammatoire est choisi parmi l'ibuprofène, le kétoprofène, l'acide acétylsalicylique, l'acide méfénamique, l'acide flufénamique, l'acide tiaprofénique, l'acide tolfénamique, le diflunisal, le piroxicam, le naproxène, le flurbiprofène, le diclofénac sodique et l'indométhacine.

4. Forme pharmaceutique solide selon la revendication 2 **caractérisée par le fait que** les sels de sodium, de potassium ou les bases inorganiques ou organiques des composés anti-inflammatoires sont utilisés comme dérivés solubles de ces composés.

5. Forme pharmaceutique solide selon la revendication 4 **caractérisée par le fait que** les dérivés sont les sels de glycine, de bétaïne, d'histidine, de lysine, d'arginine, d'ornithine, de leucine, de tyrosine sous forme racémique ou sous forme optiquement active.

6. Forme pharmaceutique solide selon la revendication 2 **caractérisée par le fait que** la substance somnifère et tranquillisante est choisie parmi le diazépam, le nitrazépam, le clonazépam et l'oxazépam.

7. Forme pharmaceutique solide selon la revendication 2 **caractérisée par le fait que** la substance antiangineuse et antihypertensive est choisie parmi le diltiazem, l'urapidil, le trapidil, le benziodarone et le dipyridamole.

8. Forme pharmaceutique solide selon la revendication 2 **caractérisée par le fait que** la substance antihistaminique ou antiasthmatique est choisie parmi la terfénadine, la chlorphéniramine, la terbutaline et des associations de ces substances.

9. Forme pharmaceutique solide selon la revendication 1 **caractérisée par le fait que** lesdits polymères sont choisis parmi le polyvinylpyrrolidone réticulé, la carboxyméthylcellulose sodique réticulée, le carboxyméthylamidon, un copolymère de méthacrylate potassique et de divinylbenzène, les alcools polyvinyliques, les amidons et leurs dérivés, la cellulose microcristalline et les dérivés de la cellulose, la bêta-cyclodextrine et les dérivés de la dextrine en général.

10. Forme pharmaceutique solide selon la revendication 9 **caractérisée par le fait que** les polymères sont présents dans la première fraction en une quantité correspondant à 1,0 à 90 % du poids de la fraction.

11. Forme pharmaceutique solide selon la revendication 10 **caractérisée par le fait que** les polymères sont présents dans la première fraction en une quantité correspondant à 20 à 70 % du poids de la fraction.

12. Forme pharmaceutique solide selon la revendication 1 **caractérisée par le fait que** les substances adjuvantes agissant comme diluants sont choisies, selon les caractéristiques hydrophiles de l'ingrédient actif, parmi l'amidon, l'amidon prégélatinisé, le phosphate de calcium, le mannitol, le lactose, le saccharose, le glucose, le sorbitol et la cellulose microcristalline.

13. Forme pharmaceutique solide selon la revendication 1 **caractérisée par le fait que** les substances adjuvantes agissant comme agglutinants sont choisies parmi les polymères ayant une puissance de liaison.

14. Forme pharmaceutique solide selon la revendication 13 **caractérisée par le fait que** les polymères sont choisis parmi la gélatine, le polyvinylpyrrolidone, la méthylcellulose, l'amidon d'encollage, l'éthycellulose, la gomme arabique et la gomme adragante.

15. Forme pharmaceutique solide selon la revendication 1 **caractérisée par le fait que** les substances adjuvantes ayant pour fonction de promouvoir la mouillabilité et la solubilisation de l'ingrédient actif sont choisies parmi les tensioactifs ioniques et non ioniques, les saponines et les stérols.

16. Forme pharmaceutique solide selon la revendication 15 **caractérisée par le fait que** la substance promouvant la mouillabilité et la solubilisation de l'ingrédient actif est contenue en une quantité correspondant à 0,01 à 25 % du poids de l'ingrédient actif.

17. Forme pharmaceutique solide selon la revendication 1 **caractérisée par le fait que** les substances adjuvantes agissant comme lubrifiants et anti-adhérants sont choisies parmi le stéarate de magnésium, l'acide stéarique, la silice colloïdale, le monostéarate de glycéryl, des polyoxy-éthylène-glycols ayant un poids moléculaire compris entre 400 et 50 000, l'huile de ricin hydrogénée, des cires et des glycérides monosubstitués, disubstitués et trisubstitués.

18. Forme pharmaceutique solide selon la revendication 1 **caractérisée par le fait que** les substances adjuvantes ont un effet effervescent et sont choisies parmi le carbonate de sodium et/ou d'autres métaux alcalins et/ou d'autres métaux alcalinaux terreux, le bicarbonate de sodium, le carbonate de sodium, la glycocolle et d'autres sels pharmaceutiquement acceptables pouvant produire une effervescence dans un milieu acide, l'acide citrique, l'acide tartrique et l'acide fumarique.

19. Forme pharmaceutique solide selon la revendication 1 **caractérisée par le fait que** les substances adjuvantes produisant l'effervescence sont choisies parmi l'acide citrique, l'acide tartrique et l'acide fumarique.

20. Forme pharmaceutique solide selon les revendications 18 et 19 **caractérisée par le fait que** les substances adjuvantes ayant un effet effervescent sont présentes en une quantité correspondant à 3 à 40 % du poids de la première fraction.

21. Forme pharmaceutique solide selon la revendication 1 **caractérisée par le fait que** la seconde fraction contient des matériaux polymériques biocompatibles naturels et/ou synthétiques ou semi-synthétiques appartenant à la classe des polymères hydrophiles gélifiants et/ou érodables capables de ralentir la libération de l'ingrédient actif de la seconde fraction.

22. Forme pharmaceutique selon la revendication 21 **caractérisée par le fait que** les polymères hydrophiles sont choisis parmi les hydroxypropylméthylcelluloses ayant un poids moléculaire compris entre 1 000 et 2 000 000 de daltons, les polymères de carboxyvinyl, les alcools polyvinyliques, les glycanes, les xanthanes, les mannanes, les galactomannanes, les scléroglucanes, les alginates, la pectine, l'amylose, ayant différents degrés de réticulation, la carboxyméthylcellulose et ses dérivés, la méthylcellulose, l'éthylcellulose et leurs sels et/ou dérivés.

23. Forme pharmaceutique selon les revendications 21 et 22 **caractérisée par le fait que** les substances polymériques peuvent être présentes en un pourcentage allant de 5 à 90 % du poids total de la fraction à libération prolongée.

24. Forme pharmaceutique selon les revendications 21 et 22 **caractérisée par le fait que** les substances polymériques peuvent être présentes en un pourcentage allant de 30 à 75 % du poids total de la fraction à libération prolongée.

25. Forme pharmaceutique solide selon la revendication 1 **caractérisée par le fait qu'**elle se présente sous la forme d'un comprimé bicouche dont chaque couche constitue l'une des fractions du composant.

26. Forme pharmaceutique solide selon la revendication 25 **caractérisée par le fait qu'**elle contient 1 à 90 % de son poids en ingrédient actif.

27. Forme pharmaceutique solide selon la revendication 25 **caractérisée par le fait qu'**elle contient de l'ibuprofène comme ingrédient actif avec une distribution dimensionnelle de moins de 20 microns.

28. Forme pharmaceutique solide selon la revendication 25 **caractérisée par le fait qu'**elle contient du laurylsulfate de sodium comme tensioactif.

29. Forme pharmaceutique solide selon la revendication 1 **caractérisée par le fait qu'**elle se présente sous la forme d'une gélule contenant un ou plusieurs comprimés contenant au moins un ingrédient actif sous une forme soluble dans l'eau et les fluides aqueux et un ou plusieurs comprimés contenant le même au moins un ingrédient identique sous forme à libération retardée et programmée.

30. Forme pharmaceutique solide selon la revendication 1 **caractérisée par le fait qu'**elle se présente sous la forme d'une gélule contenant un ou plusieurs comprimés bicouche selon la revendication 26.

31. Forme pharmaceutique solide selon la revendication 1 **caractérisée par le fait qu'**elle se présente sous la forme d'une gélule contenant au moins deux granulats différents dont au moins un contient au moins un ingrédient actif sous une forme facilement soluble dans l'eau ou les fluides aqueux et dont au moins un contient le même au moins un ingrédient actif sous une forme à libération retardée et programmée.

32. Forme pharmaceutique solide selon la revendication 31 **caractérisée par le fait que** les granulats sont sous forme agglomérée.
